(19) Europäisches Patentamt
European Patent Office
Office européen des brevets

(11) **EP 1 536 872 B1**

(12) **FASCICULE DE BREVET EUROPEEN**

(45) Date de publication et mention
de la délivrance du brevet:
**26.12.2012 Bulletin 2012/52**

(21) Numéro de dépôt: **03753661.2**

(22) Date de dépôt: **23.07.2003**

(51) Int Cl.:
***B01D 15/02*** *(2006.01)*

(86) Numéro de dépôt international:
**PCT/FR2003/002327**

(87) Numéro de publication internationale:
**WO 2004/020068 (11.03.2004 Gazette 2004/11)**

(54) **METHODE POUR OPTIMISER LE FONCTIONNEMENT D UNE UNITE DE SEPA    RATION DE XYLENES PAR CONTRE COURANT SIMULE**

VERFAHREN ZUR OPTIMIERUNG DES BETRIEBES EINER TRENNEINRICHTUNG VON XYLENEN DURCH SIMULIERTES GEGENSTROM

METHOD OF OPTIMISING THE OPERATION OF A XYLENE SEPARATION UNIT USING SIMULATED COUNTERCURRENT

(84) Etats contractants désignés:
**BE DE ES IT NL**

(30) Priorité: **28.08.2002 FR 0210658**

(43) Date de publication de la demande:
**08.06.2005 Bulletin 2005/23**

(73) Titulaire: **Institut Français du Pétrole
92852 Rueil-Malmaison Cedex (FR)**

(72) Inventeurs:
 • **COUENNE, Nicolas
  69008 Lyon (FR)**
 • **WOLFF, Luc
  69006 Lyon (FR)**

(56) Documents cités:
  **EP-A- 0 875 268    EP-A- 1 101 516
  WO-A-01/87452    US-A- 5 470 482**

**Description**

La présente invention concerne une méthode pour optimiser le fonctionnement d'un procédé de séparation des xylènes par contre courant simulé en marche hybride.

*Etat de la technique*

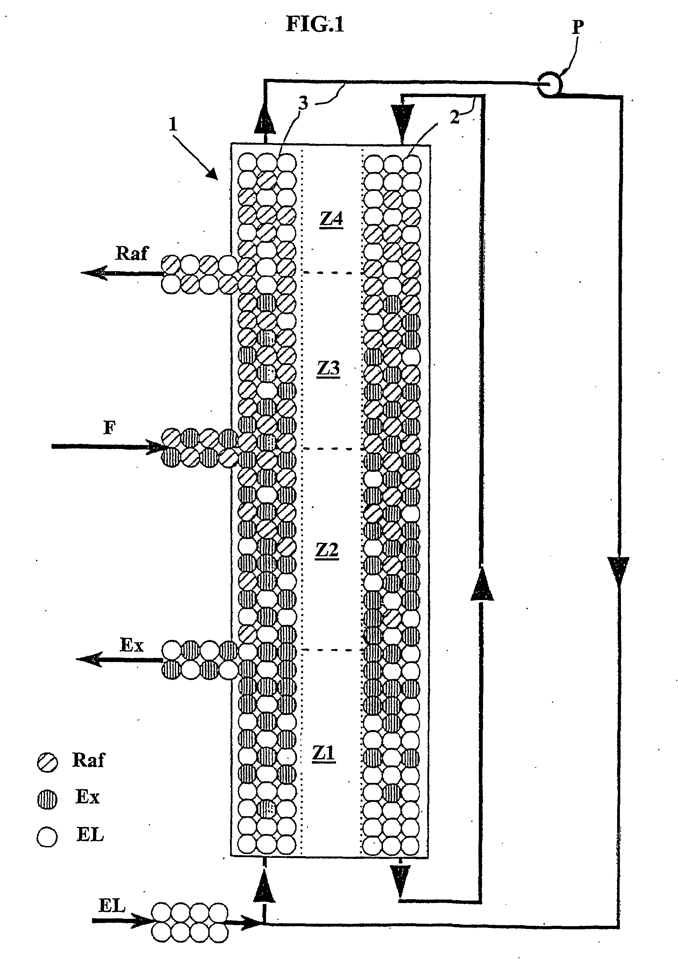

Les procédés de séparation ou fractionnement basés sur la chromatographie sont le plus souvent mis en oeuvre dans un système de séparation comportant (Fig.1) un ensemble de colonnes ou fractions de colonnes interconnectées en série, formant une boucle fermée. Un solide poreux, de granulométrie déterminée, constitue la phase stationnaire. Le mélange à séparer est introduit dans la colonne puis déplacé au moyen d'un fluide vecteur ou désorbant (EL) et les différents constituants sortent successivement selon qu'ils sont retenus plus ou moins fortement par la phase stationnaire. Le long de cette boucle sont répartis des points d'injection pour le mélange ou charge F contenant l'ensemble des constituants à séparer et le solvant ou désorbant EL, et des points d'extraction pour un extrait Ex contenant le produit que l'on cherche à valoriser dilué dans du solvant, et pour un raffinat Rf contenant tous les autres constituants. Ces points délimitent différentes zones (Z1 à Z4 par exemple). Toutes les colonnes ou fractions de colonne d'une même zone sont traversées par un débit liquide identique. Une pompe P est placée quelque part dans la boucle pour assurer la circulation du fluide dans le sens indiqué sur le schéma.

Dans un système de séparation à contre-courant réel, un profil des concentrations fixe et constant se développe où les positions des points d'injection et de soutirage restent fixes. Le solide adsorbant 3 et le liquide 2 se déplacent à contre courant. Un système d'entraînement du solide et la pompe P de recyclage, placés tous les deux à la jonction des zones Z1 et Z4, permettent de renvoyer respectivement le solide de la base vers le sommet, et le liquide inversement du sommet vers la base.

Les systèmes dits à lits mobiles simulés permettent d'échapper à une difficulté majeure inhérente aux procédés à lits mobiles vrais, celle de faire correctement circuler la phase solide sans créer d'attrition et sans augmenter considérablement la porosité de lit par rapport à celle d'un lit fixe. Pour simuler son déplacement, le solide est disposé dans un certain nombre n de lits fixes (en général, $4 \leq n \leq 24$) disposés en série et c'est le profil de concentrations que l'on déplace à vitesse sensiblement uniforme tout autour d'une boucle fermée. En pratique, le décalage successif des points d'injection et de soutirage se fait à l'aide d'une vanne rotative ou plus simplement d'un ensemble de vannes tout ou rien convenablement commandées. Ce décalage circulaire, effectué à chaque période, des différents débits liquides d'entrée-sortie dans un sens donné revient à simuler un déplacement de l'adsorbant solide dans l'autre sens.

Les systèmes de séparation utilisés pour la séparation des xylènes sont le plus souvent constitués de quatre zones principales. Il en existe également à cinq zones où une partie de l'extrait séparé du solvant est réinjectée entre le prélèvement d'extrait et l'injection de charge. D'autres encore peuvent comporter de cinq à sept zones où des fluides secondaires permettent de rincer des lignes véhiculant successivement plusieurs fluides, de manière à éviter les contaminations.

Dans la suite du texte, on désignera par :

- variables commandées, des variables qui doivent être constamment proches d'une valeur de consigne préalablement spécifiée et qui traduisent le bon fonctionnement du procédé. Il s'agit par exemple de la pureté des constituants d'un extrait, du rendement de l'unité de séparation pour un certain constituant etc.;

- variables opératoires, des variables pouvant être modifiées par l'opérateur, telles que les débits ou encore la période de commutation de vannes permettant de simuler le déplacement des lits, etc.;

- variables de commande, des variables qui agissent principalement sur une seule zone par exemple sur la partie du profil de concentration contenue dans une zone. Ces variables de commande sont déterminées par l'algorithme de contrôle, et sont traduites en variables opératoires.

On rappelle que le but d'un système de contrôle avancé fonctionnant sur un procédé est de calculer une loi de commande (ensemble des valeurs des variables opératoires au cours du temps) pour :

- asservir le fonctionnement i.e. calculer une loi de commande capable d'assurer la transition entre deux valeurs distinctes d'une ou de plusieurs variables commandées choisies a priori; et

- réguler le fonctionnement i.e. calculer une loi de commande capable de compenser au mieux (par avance ou au moins asymptotiquement) toutes les perturbations extérieures agissant sur le processus afin que les variables commandées choisies a priori gardent une valeur quasi constante.

Dans le cas d'une unité de séparation par contre courant simulé, la régulation peut également compenser des perturbations dues à une évolution dans le temps des paramètres thermodynamiques et géométriques de l'adsorbant (bien entendu pour une détérioration limitée des propriétés de l'adsorbant).

Ces objectifs sont remplis avec le procédé de contrôle automatique qui est basé soit sur une technique de type "boite noire" soit sur une approche plus maîtrisée permise par une modélisation non linéaire du processus de séparation

Par le brevet EP 875.268 (US 5.902.486) du demandeur, on connaît un procédé de contrôle automatique d'un système de séparation à lits mobiles simulés, de constituants d'un mélange de fluides en circulation, notamment d'hydrocarbures aromatiques, pouvant présenter des variations notables de débit ou de la qualité de la charge. Le contrôle du processus (de type multivariable non linéaire effectué à partir d'un modèle de connaissance ou linéaire au voisinage d'un point de fonctionnement donné effectué à partir de modèles de représentation entreé/sortie) est réalisé avec un certain nombre de mesures de variables en une pluralité de points de mesure le long de la boucle (des concentrations et des débits par exemple) et de mesures caractéristiques des fluides injectés et soutirés. On détermine à partir de valeurs courantes de variables commandées (pureté des constituants, rendement du système etc.) dépendant des variables mesurées, des ratios indicatifs respectivement du rapport dans chacune des différentes zones, entre les débits de fluides et les débits simulés de matière adsorbante. A partir de ces ratios, on détermine des valeurs à donner aux variables opératoires pour amener ou ramener les variables commandées jusqu'à des valeurs de consigne déterminées. Si l'on dispose par exemple de quatre variables de commande indépendantes, les quatre ratios de chacune des zones, il faut déterminer quatre variables commandées.

Le procédé de contrôle comporte un algorithme de calcul qui détermine les ratios à partir des mesures effectuées et nécessaires aux calculs des variables commandées. Ce calcul peut être réalisé de deux manières complètement différentes : soit en utilisant un modèle physique non linéaire de l'unité de séparation en lit mobile vrai, soit en utilisant une association de modèles linéaires monovariables chacun représentant le comportement d'une sortie (une variable commandée) vis à vis d'une entrée (une variable de commande), sachant que l'association de ces modèles linéaires est souvent qualifiée de "boîte noire" dans la terminologie spécialisée.. La détermination de ces modèles simples s'effectue à partir d'un jeu de mesures expérimentales obtenu sur le procédé fonctionnant dans un état proche de son état stable prévu.

Dans sa version développée pour séparer les xylènes, le procédé est utilisé pour purifier le paraxylène présent dans des charges contenant en majorité des xylènes mais également des C9 aromatiques et des paraffines en quantité limitée. Il se décline sous deux versions : la version dite «standalone» qui permet d'atteindre une pureté supérieure à 99.80% et la version "hybride" qui est dimensionnée pour atteindre une pureté de l'ordre de 95.00%. Cette dernière version du procédé, décrite par exemple dans le brevet EP 531.191, est commercialisée avec l'ajout d'un procédé de cristallisation permettant d'atteindre la haute pureté désirée. Les unités fonctionnant en mode hybride sont constituées d'au moins 12 colonnes alors qu'il y a en au moins 24 pour les modes de fonctionnement en "standalone".

Que ce soit un procédé de contrôle automatique non linéaire ou de type boite noire, il s'agit de calculer, à partir de la mesure de concentrations de certains constituants nécessaires au calcul de variables commandées, les ratios (Rk) indicatifs respectivement du rapport dans chacune des différentes zones, entre les débits de fluides (Qk) et le débit simulé de matière adsorbante (Qs) de façon à amener ou ramener les variables commandées jusqu'à des valeurs de consignes déterminées. Dans une seconde étape, les valeurs des ratios ainsi déterminées, seront transformées en variables opératoires appliquées au processus en utilisant les formules de conversion.

Le procédé de contrôle permet donc d'amener l'unité de séparation à un point de fonctionnement où l'on amène jusqu'à des valeurs spécifiées les quatre paramètres suivants:

1. la pureté du paraxylène dans l'extrait défini comme suit $\text{Purete} = \dfrac{Px^{extrait}}{Px^{extrait} + \text{Imp}^{extrait}}$, où

$Px^{extrait}$ est la concentration du paraxylène dans extrait, et
$\text{Imp}^{extrait}$ représente l'ensemble des impuretés dans l'extrait.
La détermination de cette variable commandée nécessite la mesure en ligne dans l'extrait d'une part, de la concentration de paraxylène et d'autre part, de l'ensemble des autres constituants ;

2. le rendement en paraxylène de l'unité défini comme suit

$$\text{Rendement} = 1 - \frac{Q_{raffinat}\,Px^{raffinat}}{Q_{raffinat}\,Px^{raffinat} + Q_{extrait}\,Px^{extrait}}, \text{ où}$$

$Px^{extrait}$ et $Px_{raffinat}$ représentent respectivement la concentration de paraxylène dans l'extrait et dans le raffinat, et

3

$Q_{raffinal}$ et $Q_{extrait}$ représentent respectivement les débits de raffinat et d'extrait.

La détermination de cette variable commandée nécessite la mesure en ligne de la concentration de paraxylène dans l'extrait et dans le raffinat ainsi que la mesure des débits d'extrait et de raffinat.

3. La quantité d'éthylbenzènze dans l'extrait notée $Eb_{Extrait}$.

La détermination de cette variable commandée nécessite la même mesure en ligne que développée pour le point 1.

4. La quantité de paraxylène en un point de la zone 1 notée $Px_{Zone1}$.

La détermination de cette variable commandée nécessite le développement d'un point de mesure spécifique en zone 1 c'est à dire comprise entre l'injection de solvant et le soutirage de d'extrait.

Si les deux premières variables commandées correspondent clairement à des objectifs de production, les deux dernières sont directement liées à l'objet de la présente invention c'est à dire à l'optimisation du fonctionnement de l'unité.

Pour contrôler l'unité de séparation, on a besoin de mesures de concentration en trois points distincts de la boucle. Ces mesures sont faites par chromatographie ou par spectrométrie Raman tel que celui décrit dans le brevet FR 2.699.917 (US 5.569.808) du demandeur. Pour calculer en ligne la pureté et le rendement et pour mesurer la quantité d'éthylbenzène dans l'extrait, il est nécessaire de disposer de deux mesures qui fournissent les concentrations des différents constituants dans l'extrait et dans le raffinat. La dernière sortie $Px_{Zone1}$ nécessite un point de mesure en zone Z1. La durée d'une analyse est de quelques secondes (par spectrométrie Raman) à 20 minutes (par chromatographie). Compte tenu des temps de réponse de l'unité (entre 4 et 8 heures), la qualité de la commande du procédé n'est pas affectée par le temps d'analyse, s'il reste inférieur à l'heure.

La conversion des variables de commande (les ratios Rk) en variables opératoires "classiques" est toujours possible, en dehors des contraintes physiques réelles d'application liées au dimensionnement du procédé et de son équipement, car il existe une relation biunivoque entre elles, condition nécessaire pour rendre le système de séparation parfaitement contrôlable.

On sait que le fonctionnement d'un système de séparation par contre courant simulé est quasi-identique à celui d'un système à lits mobiles vrais si, pour celui-ci, les débits circulant à contre-courant du débit liquide principal vérifient les relations d'équivalence décrites dans le brevet EP 875.268 précité.

Les variables de commande (les ratios Rk) sont déterminées par rapport à ces équivalences comme les rapports adimensionnels entre les débits liquides principaux dans chacune des zones et le débit solide qui est constant dans toute l'unité de séparation :

$$R_k = \frac{Q_k}{Q_s}$$

Le choix de ces ratios découle de l'écriture des équations de bilan matière du modèle d'une unité de séparation de lit mobile vrai à l'état stationnaire sur une portion de colonne que l'on discrétise. Le nombre de ratios est égal au nombre de zones constituant l'unité, chaque zone étant caractérisée par un débit liquide principal distinct des zones adjacentes.

La méthode selon l'invention

La méthode selon l'invention permet d'optimiser le fonctionnement d'une unité de séparation de composants d'une charge comprenant une boucle de séparation constituée par l'interconnexion d'un ensemble de lits contenant un matériau solide adsorbant formant plusieurs zones délimitées par des points d'injection d'une charge et d'un solvant et des points d'extraction hors de la boucle d'un extrait contenant un premier composant de la charge, et d'un raffinat, des moyens de permutation des points d'injection et des points d'extraction permettant de simuler le déplacement des lits à contre-courant et des moyens de mesure de variables opératoires.

Elle comporte l'utilisation d'un algorithme de contrôle pour amener l'unité de séparation à un point de fonctionnement où la pureté du premier composant dans l'extrait (tel que le paraxylène par exemple) et le rendement de l'unité de séparation dans la production de ce premier composant, sont portées à des valeurs spécifiées.

Suivant un premier mode de mise en oeuvre, pour une valeur donnée de la concentration dans l'extrait, d'un deuxième composant de la charge (tel que de l'éthylbenzène par exemple), on règle la valeur de consigne de la concentration du premier composant dans une zone située entre le point d'injection du solvant et le point d'extraction de l'extrait, de façon à minimiser le taux de solvant relativement à la charge.

Le réglage de la valeur de consigne de la concentration est obtenue avantageusement au moyen d'un optimiseur monovariable.

Le réglage de la valeur de consigne de la concentration du deuxième composant dans l'extrait est fait de préférence, pour maximiser la capacité de l'unité de séparation, dans les limites de stabilité de fonctionnement de la dite unité.

Suivant un autre mode de mise en oeuvre, on règle la valeur de consigne de la concentration dans l'extrait du deuxième composant de la charge, pour obtenir une maximisation de la capacité de l'unité de séparation dans les limites de stabilité de fonctionnement de la dite unité.

De préférence la valeur de consigne de la concentration dans l'extrait du deuxième composant doit se situer dans un intervalle compris entre 0.02% et 2%.

## Présentation succincte des figures

Les caractéristiques et avantages de la méthode selon l'invention, apparaîtront plus clairement à la lecture de la description ci-après d'un exemple non limitatif de réalisation, en se référant aux dessins annexés où :

- la figure 1 montre schématiquement une unité de séparation à quatre zones avec des points d'injection et de soutirage intercalés ;

- les figures 2a, 2b montrent respectivement la variation du taux de solvant (S/F) et du débit de recyclage moyen (MR) en fonction de $Px_{Zone1}$ et $Eb_{Extrait}$ ; et

- la figure 3 montre les profils de concentration (C%) de Px, Eb, Mox le long de la boucle de séparation aux points de fonctionnement optimaux au sens de (S/F) à pureté et rendement constants.

## Description détaillée

La méthode selon l'invention permet d'optimiser le fonctionnement d'une unité de séparation des xylènes en mode hybride à quatre zones, amenée préalablement par application du procédé de contrôle objet du brevet EP 875.268 précité, à un point de fonctionnement où deux variables commandées caractérisant directement la qualité et la production du produit, i.e. la pureté du paraxylène dans l'extrait et le rendement en paraxylène définis plus haut, sont portées à des valeurs spécifiées.

L'optimisation est conduite en utilisant l'algorithme de contrôle qui y est décrit. Pour optimiser le fonctionnement, comme on dispose de quatre variables de commande indépendantes (les quatre ratios de chacune des zones) et qu'il faut déterminer deux autres variables commandées, on va agir sur :

- la concentration d'éthylbenzène dans l'extrait notée $Eb_{Extrait}$ que l'on peut déterminer avantageusement en utilisant le moyen de mesure mise en oeuvre pour la mesure de la pureté; et
- la concentration en paraxylène en un point donné de la zone Z1 (définie comme étant la zone comprise entre l'injection du solvant et le prélèvement de l'extrait) notée $Px_{Zone1}$, qui peut être déterminée par une la mesure de la concentration en paraxylène en un point donné de la zone Z1.

La concentration d'éthylbenzène dans l'extrait permet de caractériser la position du profil d'éthylbenzène dans la zone Z2. La concentration en paraxylène en un point donné de la zone Z1, est déterminée pour contrôler le flux de paraxylène en aval du point d'injection de solvant.

On utilise les deux dernières variables commandées pour maximiser ou minimiser une fonctionnelle que l'on a défini en fonction d'objectifs de production (coût économique par exemple) que l'on se fixe a priori pour ce genre d'unité de séparation comme par exemple :

1. La minimisation du taux de solvant défini comme le rapport entre le débit de solvant et le débit de charge.

2. La maximisation du débit de charge.

3. La minimisation du débit de recyclage moyen défini comme suit :

$$Q_{moyen} = \frac{1}{Nb_{Colonne}} \sum_{i=1}^{NbZone} l_i\, Q_i$$

où

$Nb_{Zone}$      représente le nombre de zones de l'unité

$Nb_{Colonne}$      représente le nombre de colonnes total

$l_i$      représente le nombre de colonnes dans chaque zone

$Q_i$      représente le débit liquide dans chaque zone

Dans ce qui suit, on va s'intéresser uniquement aux points 1 et 2 car l'optimisation du point 3 est équivalente à celle du point 2.

On optimise ensemble les points 2 et 3 car ils sont reliés par l'homogénéité du système d'équations reliant les ratios et les variables opératoires. Toute augmentation d'un des débits peut être compensée par une augmentation semblable des autres débits et une diminution dans la même proportion de la période de permutation des vannes. La maximisation du débit de charge peut donc être associée directement à la minimisation du débit de recyclage dans le sens où plus le débit de recyclage est faible et plus la marge pour augmenter le débit de charge est grande. Ces considérations « idéales » sont limitées en pratique par l'hydrodynamique comme par exemple l'augmentation de la dispersion axiale qui varie de façon quadratique avec la vitesse du fluide dans la porosité externe.

Le point 1 est relatif à l'excès de consommation de solvant dont le coût de distillation est élevé. L'optimisation de l'unité de séparation, au sens des points 1 et 2, est obtenue par un réglage judicieux des sorties PxZone1 et EbExtrait.

**Résultats de simulation**

Nous montrons, avec la simulation, que ces objectifs d'optimisation ne sont pas indépendants et qu'il existe un minimum absolu pour le taux de solvant dépendant à la fois de $Px_{Zone1}$ et $Eb_{Extrait}$. Nous verrons, avec les résultats expérimentaux, que nous recommandons une marche sous optimale (pour garantir la stabilité de l'unité) permettant néanmoins de gagner significativement en taux de solvant et en capacité.

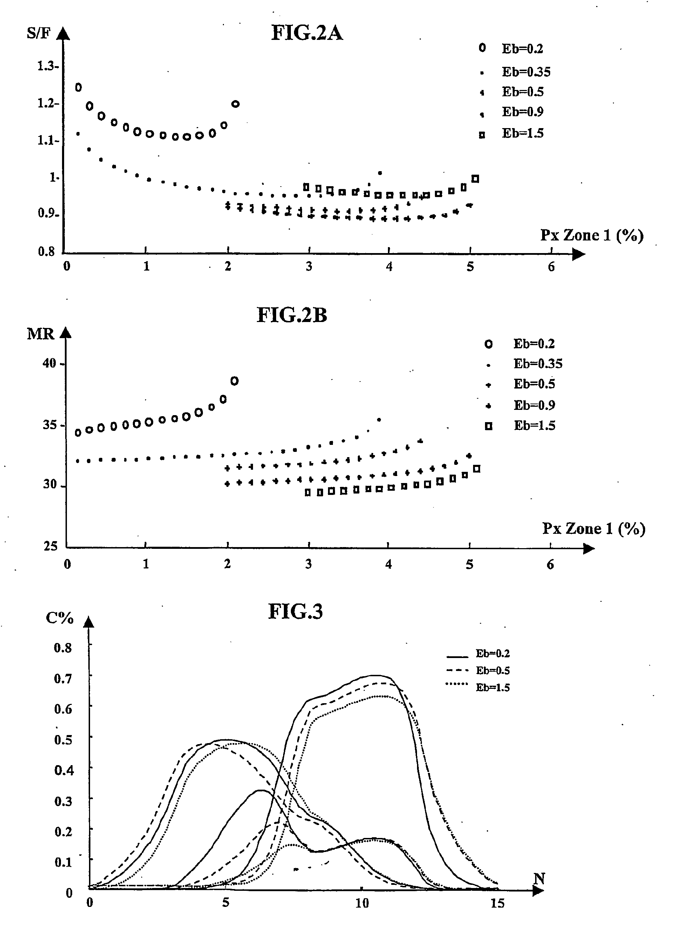

Les résultats obtenus en simulation sont résumés sur les graphiques de la Fig.2.

Les courbes représentent la variation du taux de solvant (S/F) et du débit de recyclage moyen tels que définis ci-dessus, en fonction de la valeur de $Px_{Zone1}$ (en abscisse) pour une valeur de $Eb_{Extrait}$ donnée, à pureté constante, à rendement constant et à débit de charge constant.

Les variations de (S/F) sont strictement concaves à la fois par rapport à $Px_{Zone1}$ (évident au vu des courbes) mais aussi par rapport à $Eb_{Extrait}$ puisque la courbe ($Eb_{Extrait}$ = 1.5%) est située au dessus des courbes ($Eb_{Extrait}$ = 0.5%) et ($Eb_{Extrait}$ = 0.9%) et en dessous de la courbe ($Eb_{Extrait}$ = 0.35%). Il y a donc un minimum absolu pour (S/F) dont la valeur n'est pas représentée sur ces courbes.

La variation du débit de recyclage moyen (MR) par rapport à $Px_{Zone1}$ et à $Eb_{Extrait}$ est strictement monotone croissante. La valeur du débit de recyclage moyen (MR) décroît lorsque $Eb_{Extrait}$ augmente et croît lorsque la valeur de $Px_{Zone1}$ augmente, toutes les autres spécifications étant constantes par ailleurs.

Les résultats ci-dessus montrent que l'optimisation simultanée des points 1 et 2 (i.e. minimiser S/F et maximiser le débit de charge) n'est pas possible. Il existe un minimum absolu pour (S/F) qui ne correspond pas au minimum possible susceptible d'être atteint par le débit de recyclage moyen.

**Résultats expérimentaux**

Les résultats expérimentaux, obtenus sur l'unité pilote, confirment les tendances mises en évidence avec la simulation.

Dans l'exemple qui suit, seul l'effet de $Px_{Zone1}$ est présenté car l'influence de $Eb_{Extrait}$ sur le fonctionnement de l'unité est nettement plus évident et n'a pas nécessité d'expériences spécifiques.

La comparaison des 2 points stationnaires suivants :

| Point stationnaire n°1 | Point stationnaire n°2 |
|---|---|
| Pureté = 95% | Pureté = 95% |
| Rendement = 96% | Rendement = 96% |
| EbExtrait = 0.06% | EbExtrait = 0.06% |
| PxZone1 = 4% | PxZone1 = 1.8% |
| Qcharge = 68cc/min | Qcharge = 78cc/min |
| Qrecyclage = 379cc/min | Qrecyclage = 379cc/min |
| S/F= 1.15 | S/F=1.1 |

montre que pour les mêmes spécifications de pureté et de rendement on peut passer 10cc/min de charge en plus, obtenir un taux de solvant légèrement plus faible et conserver le même débit de recyclage en changeant seulement la consigne de PxZone1.

*Caractéristiques des points optimaux obtenus*

Afin de comparer les différents points optimaux (au sens de la minimisation de (S/F)) obtenus ci-dessus en simulation, on trace sur le graphique suivant l'ensemble des profils de concentration C le long des colonnes de la boucle de séparation pour trois valeurs significatives de $Eb_{Extrait}$.

Les traits verticaux du graphique de la Fig.3 représentent respectivement de gauche à droite : le point de soutirage Vex de l'extrait, le point d"injection de la charge $V_F$ et le soutirage du raffinat $V_{RAF}$.

L'ensemble des profils recule lorsque le débit de recyclage moyen diminue. Près du point de soutirage Vex de l'extrait les différents profils sont assez distincts car les proportions entre les impuretés dans l'extrait varient sensiblement puisque l'une d'entre elles (l'éthylbenzène) est le paramètre caractérisant ces simulations. Dans la zone Z3, comprise entre l'injection de la charge VF et le soutirage du raffinat $V_{RAF}$, les profils des différentes simulations sont assez « proches ». Leur forme caractérise bien les modes de fonctionnement optimaux de cette unité de séparation à savoir que le soutirage du raffinat est toujours situé à la base du profil de paraxylène en zone Z3. Les différences obtenues sur la valeur de la concentration de paraxylène au niveau du soutirage du raffinat s'expliquent par une valeur constante du rendement pour toutes les simulations. Les petites variations de la valeur de la concentration en Px compensent les variations de la valeur du débit de raffinat propre à chaque simulation.

**Optimisation et conduite de l'unité de séparation**

L'examen des résultats obtenus en simulation complétés par les résultats expérimentaux nous permettent d'énoncer les règles suivantes pour la conduite optimale de l'unité de séparation en mode hybride au sens de la minimisation du rapport (S/F) et de la maximisation de la capacité i.e. le réglage de l'unité de séparation de telle façon qu'elle puisse traiter potentiellement le maximum de charge. La mise en application de cette stratégie d'optimisation n'est concevable en pratique qu'avec l'utilisation d'un algorithme de contrôle comme par exemple celui présenté dans le brevet EP 531.191 précité.

Un choix judicieux des consignes $Px_{Zone1}$ et $Eb_{Extrait}$ permet d'atteindre l'optimum de la conduite de l'unité de séparation au sens de la minimisation de (S/F) pour une pureté et un rendement donnés.

Compte tenu que l'on sait par les études effectuées en simulation que l'on se situe toujours (Fig.2) sur des courbes strictement concaves et qu'en faisant varier $Px_{Zone1}$, on se déplacera sur le long de telles courbes, et aussi du fait des contraintes imposées à la consigne $Eb_{Extrait}$ pour garantir une maximisation de la capacité tout en garantissant la stabilité de l'unité, un optimiseur monovariable très simple de type simplex bien connu des spécialistes, peut être utilisé pour rechercher automatiquement en ligne itérativement la valeur de la consigne optimale de $Px_{Zone1}$. Un tel optimiseur est pratique car il peut fonctionner sans avoir à calculer de gradients numériques. L'avantage est de limiter le nombre d'évaluations de la fonction coût qui dans le cas de la présente méthode, correspondent à autant de points de fonctionnement de l'unité de séparation potentiellement hors du point de fonctionnement optimal.

Dans le cadre de cette application, l'optimisation se fera avec la génération de triangles dans le plan, chaque sommet du triangle étant une solution potentielle. À chaque étape de la recherche de l'optimum, un nouveau point, dans le triangle actuel, ou proche de celui-ci, sera produit. La valeur de la fonction au nouveau point est comparée aux valeurs de la fonction aux sommets du simplex et, habituellement, un des sommets est remplacé par le nouveau point, donnant un nouveau simplex et une meilleure estimation de la fonction coût. Cette étape est répétée jusqu'à ce que le diamètre du simplex soit plus petit que la tolérance choisie.

Un choix judicieux de la consigne $Eb_{Extrait}$ permet de garantir la maximisation de la capacité de l'unité mais avec les deux remarques suivantes :

1. La valeur optimale de cette consigne ne peut être atteinte car, dans ce cas, le régime de fonctionnement atteint par l'unité n'est pas stable. En effet, une très faible diminution de débit de recyclage engendre une quantité de plus en grande d'éthylbenzène accumulée dans la colonne, ce qui rend la conduite de l'unité de séparation difficile car la dynamique d'augmentation de la quantité d'éthylbenzène dans l'extrait est beaucoup plus rapide que la dynamique de diminution.

2. La consigne optimum pour $Eb_{Extrait}$ en terme de capacité ne coïncide pas avec celle recherchée pour optimiser (S/F) qui est plus faible.

Compte tenu des deux remarques précédentes, il est bien préférable de privilégier la stabilité de l'unité de séparation et de choisir dans ce cadre une consigne $Eb_{Extrait}$ garantissant une maximisation de la capacité, sans chercher à atteindre l'optimal possible. Dans cette configuration, l'optimisation de (S/F) se ramène bien à celle exposée ci-dessus.

Compte tenu des résultats expérimentaux et de simulation, la valeur de $Eb_{Extrait}$ choisie dépend directement de la performance du système de contrôle de l'unité de séparation et de la valeur de la pureté qui est choisie.

La raideur du profil (tous les profils des différentes simulations sont semblables) en zone Z3 et la position spécifique du soutirage du raffinat Raf à la base de ce front, indiquent une grande sensibilité de la valeur du rendement aux petites variations de débit ou toutes autres perturbations engendrant une variation de la position du profil. Cette grande sensibilité du rendement indique que la conduite d'une unité à son point optimal est difficile manuellement.

## Revendications

1. Méthode pour optimiser le fonctionnement d'une unité de séparation de composants d'une charge, comprenant une boucle de séparation constituée par l'interconnexion d'un ensemble de lits contenant un matériau solide adsorbant formant plusieurs zones délimitées par des points d'injection d'une charge (F) et d'un solvant (S) et des points d'extraction hors de la boucle d'un extrait (Ex) contenant un premier composant (Px) de la charge, et d'un raffinat (Raf), des moyens de permutation des points d'injection et des points d'extraction permettant de simuler le déplacement des lits à contre-courant et des moyens de mesure de variables opératoires, comportant l'utilisation d'un algorithme de contrôle pour amener l'unité de séparation à un point de fonctionnement où la pureté du premier composant dans l'extrait et le rendement de l'unité de séparation dans la production de ce premier composant, sont portées à des valeurs spécifiées, **caractérisée en ce que**, pour une valeur donnée de la concentration ($Eb_{Extrait}$) dans l'extrait d'un deuxième composant (Eb) de la charge, on règle manuellement la valeur de consigne de la concentration ($Px_{Zone1}$) du premier composant dans une zone (Z1) située entre le point d'injection du solvant et le point d'extraction de l'extrait, de façon à minimiser le taux (S/F) de solvant relativement à la charge.

2. Méthode selon la revendication 1, **caractérisée en ce que** l'on règle la dite valeur de la concentration ($Eb_{Extrait}$), pour maximiser la capacité de l'unité de séparation, dans les limites de stabilité de fonctionnement de la dite unité.

3. Méthode pour optimiser le fonctionnement d'une unité de séparation de composants d'une charge comprenant une boucle de séparation constituée par l'interconnexion d'un ensemble de lits contenant un matériau solide adsorbant formant plusieurs zones délimitées par des points d'injection d'une charge (F) et d'un solvant (S) et des points d'extraction hors de la boucle d'un extrait (Ex) contenant un premier composant (Eb) de la charge, et d'un raffinat (Raf), des moyens de permutation des points d'injection et des points d'extraction permettant de simuler le déplacement des lits à contre-courant et des moyens de mesure de variables opératoires, comportant l'utilisation d'un algorithme de contrôle pour amener l'unité de séparation à un point de fonctionnement où la pureté du premier composant dans l'extrait et le rendement de l'unité de séparation dans la production de ce premier composant, sont portées à des valeurs spécifiées, **caractérisée en ce qu'**on règle la valeur de consigne de la concentration ($Eb_{Extrait}$) dans l'extrait d'un deuxième composant (Eb) de la charge, pour obtenir une maximisation de la capacité de l'unité de séparation dans les limites de stabilité de fonctionnement de la dite unité de séparation.

4. Méthode selon la revendication 1 ou 2, **caractérisée en ce que** l'on règle la dite valeur de consigne de la concentration ($Px_{Zone1}$) au moyen d'un optimiseur monovariable.

5. Méthode selon la revendication 3, **caractérisée en ce que** l'on règle la dite valeur de consigne de la concentration ($Eb_{Extrait}$) dans l'extrait dans l'intervalle compris entre 0.02% et 2%.

6. Méthode selon l'une des revendications précédentes, **caractérisée en ce que** le premier composant et le deuxième composant de la charge sont respectivement le paraxylène et l'éthylbenzène.

## Claims

1. A method of optimizing operation of a unit intended for separation of the constituents of a feed, comprising a separation loop consisting of the interconnection of a series of beds containing a solid adsorbent material forming several zones delimited by feed (F) and solvent (S) injection points and extraction points for discharge, out of the loop, of an extract (Ex) containing a first constituent (Px) of the fccd, and of a raffinate (Raf), injection points and extraction points switching means allowing to simulate countercurrent displacement of the beds and means for measuring operating variables, comprising using a control algorithm for bringing the separation unit to a working point where the purity of the first constituent in the extract and the yield of the separation unit as regards production of this first constituent are brought to specified values, **characterized in that**, for a given value of concentration ($Eb_{extract}$), in the extract, of a second constituent (Eb) of the feed, the set value of the concentration ($Px_{zone1}$) of the first constituent is adjusted manually in a zone (Z1) located between the solvent injection point and the extract extraction point so as to minimize the solvent/feed ratio (S/F).

2. A method as claimed in claim 1, **characterized in that** said concentration value ($Eb_{extract}$) is adjusted so as to maximize the capacity of the separation unit within the operating stability limits of said unit.

3. A method of optimizing operation of a unit intended for separation of the constituents of a feed, comprising a separation loop consisting of the interconnection of a series of beds containing a solid adsorbent material forming several zones delimited by feed (F) and solvent (S) injection points and extraction points for discharge, out of the loop, of an extract (Ex) containing a first constituent (Eb) of the feed, and of a raffinate (Raf), injection points and extraction points switching means allowing to simulate countercurrent displacement of the beds and means for measuring operating variables, comprising using a control algorithm for bringing the separation unit to a working point where the purity of the first constituent in the extract and the yield of the separation unit as regards production of this first constituent are brought to specified values, **characterized in that** the set value of concentration ($Eb_{extract}$), in the extract, of a second constituent (Eb) of the feed, is adjusted so as to obtain maximization of the capacity of the separation unit within the operating stability limits of said separation unit.

4. A method as claimed in claim 1 or 2, **characterized in that** said set value of concentration ($Px_{zone1}$) is adjusted by means of a monovariable optimizer.

5. A method as claimed in claim 3, **characterized in that** said set value of concentration ($Eb_{extract}$) in the extract is adjusted in the range between 0.02 % and 2 %.

6. A method as claimed in any one of the previous claims, **characterized in that** the first constituent and the second constituent of the feed are paraxylene and ethylbenzene respectively.


**Patentansprüche**

1. Verfahren zur Optimierung des Betriebs einer Einheit zum Abtrennen von Bestandteilen einer Charge, umfassend eine Abtrennschleife, die dadurch entsteht, dass eine Gesamtheit von Betten miteinander verbunden wird, welche ein absorbierendes Feststoffmaterial enthalten, das mehrere Bereiche bildet, welche durch Punkte zum Einleiten einer Charge (F) und eines Lösemittels (S) und Punkte zur Entnahme eines Extraktes (Ex), der einen ersten Bestandteil (Px) der Charge enthält, und eines Raffinats (Rat) aus der Schleife abgegrenzt ist, Mittel zur Permutation der Einleitungspunkte und der Entnahmepunkte, die es ermöglichen, die Verschiebung der Betten in Gegenstromrichtung zu simulieren, und Mittel zur Messung von Betriebsvariablen, wobei es die Verwendung eines Steueralgorithmus aufweist, der die Abtrenneinheit einem Betriebspunkt zuführt, an welchem die Reinheit des ersten Bestandteils im Extrakt und die Ausbeute der Abtrenneinheit bei der Produktion dieses ersten Bestandteils spezifische Werte erreichen, **dadurch gekennzeichnet, dass** für einen gegebenen Wert der Konzentration im Extrakt ($Eb_{extrait}$), welche ein zweiter Bestandteil (Eb) der Charge aufweist, der Sollwert der Konzentration ($Px_{Zone1}$) des ersten Bestandteils in einem Bereich (Z1), welcher sich zwischen dem Einleitungspunkt des Lösemittels und dem Entnahmepunkt des Extrakts befindet, manuell derart eingestellt wird, dass der Lösemittelgehalt bezogen auf die Charge (S/F) möglichst gering ist.

2. Verfahren nach Anspruch 1, **dadurch gekennzeichnet, dass** der Konzentrationswert ($Eb_{Extrait}$) derart eingestellt wird, dass die Kapazität der Abtrenneinheit möglichst groß ist, innerhalb der Grenzen eines stabilen Betriebs der Einheit.

3. Verfahren zur Optimierung des Betriebs einer Einheit zum Abtrennen von Bestandteilen einer Charge, umfassend eine Abtrennschleife, die dadurch entsteht, dass eine Gesamtheit von Betten miteinander verbunden wird, welche ein absorbierendes Feststoffmaterial enthalten, das mehrere Bereiche bildet, welche durch Punkte zum Einleiten einer Charge (F) und eines Lösemittels (S) und Punkte zur Entnahme eines Extraktes (Ex), der einen ersten Bestandteil (Eb) der Charge enthält, und eines Raffinats (Raf) aus der Schleife abgegrenzt ist, Mittel zur Permutation der Einleitungspunkte und der Entnahmepunkte, die es ermöglichen, die Verschiebung der Betten in Gegenstromrichtung zu simulieren, und Mittel zur Messung von Betriebsvariablen, wobei es die Verwendung eines Steueralgorithmus aufweist, der die Abtrenneinheit einem Betriebspunkt zuführt, an welchem die Reinheit des ersten Bestandteils im Extrakt und die Ausbeute der Abtrenneinheit bei der Produktion dieses ersten Bestandteils spezifische Werte erreichen, **dadurch gekennzeichnet, dass** der Sollwert der Konzentration im Extrakt ($Eb_{Extrait}$) eines zweiten Bestandteils (Eb) der Charge derart eingestellt wird, dass innerhalb der Grenzen eines stabilen Betriebs der Einheit eine möglichst große Kapazität der Abtrenneinheit erzielt wird.

4. Verfahren nach den Ansprüchen 1 oder 2, **dadurch gekennzeichnet, dass** der Sollwert der Konzentration ($Px_{Zone1}$) mittels einer monovariablen Optimierungseinrichtung eingestellt wird.

5. Verfahren nach Anspruch 3, **dadurch gekennzeichnet, dass** der Sollwert der Konzentration ($Eb_{Extrait}$) im Extrakt derart eingestellt wird, dass er innerhalb des Intervalls von 0,02 % bis 2 % liegt.

6. Verfahren nach einem der vorhergehenden Ansprüche, **dadurch gekennzeichnet, dass** es sich bei dem ersten Bestandteil und dem zweiten Bestandteil der Charge um para-Xylol beziehungsweise Ethylbenzol handelt.

## FIG.1

Raf

F

Ex

⊘ Raf

⊞ Ex

○ EL

EL

Z4

Z3

Z2

Z1

1

2

3

P

## FIG.2A

S/F

1.3-
1.2-
1.1-
1-
0.9-
0.8

O  Eb=0.2
•  Eb=0.35
◂  Eb=0.5
◂  Eb=0.9
▫  Eb=1.5

Px Zone 1 (%)

0       1       2       3       4       5       6

## FIG.2B

MR

40

35

30

25

O  Eb=0.2
•  Eb=0.35
✦  Eb=0.5
♦  Eb=0.9
▫  Eb=1.5

Px Zone 1 (%)

0       1       2       3       4       5       6

## FIG.3

C%

0.8
0.7
0.6
0.5
0.4
0.3
0.2
0.1
0

——  Eb=0.2
- - -  Eb=0.5
······  Eb=1.5

N

0          5          10          15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 875268 A
- US 5902486 A
- EP 531191 A
- FR 2699917
- US 5569808 A